# EUROPÄISCHE PATENTSCHRIFT

(11) **EP 2 663 544 B1**
(45) Veröffentlichungstag und Bekanntmachung des Hinweises auf die Patenterteilung: **24.12.2014**
(21) Anmeldenummer: 12700264.0
(22) Anmeldetag: 06.01.2012
(51) Int. Cl.: C07C 209/36, C07C 211/46

(54) **VERFAHREN ZUR HERSTELLUNG AROMATISCHER AMINE**
PROCESS FOR PREPARING AROMATIC AMINES
PROCÉDÉ DE PRODUCTION D'AMINES AROMATIQUES

(30) Priorität: 11.01.2011 DE 102011002497
(43) Veröffentlichungstag der Anmeldung: 20.11.2013
(73) Patentinhaber: Bayer Intellectual Property GmbH, 40789 Monheim am Rhein (DE)
(72) Erfinder: MERKEL, Michael, 40223 Düsseldorf (DE); WILKE, Karl-Heinz, 47447 Moers (DE); LEHNER, Peter, Houston, TX 77005 (US); LAGO, Andre, 22607 Hamburg (DE); DIETERICH, Erwin, 50769 Köln (DE)
(74) Vertreter: BIP Patents
(86) Internationale Anmeldenummer: PCT/EP2012/050161
(87) Internationale Veröffentlichungsnummer: WO 2012/095356

(56) Entgegenhaltungen:
- EP-A1- 1 524 259
- DE-A1- 2 849 002
- GB-A- 1 452 466

## Beschreibung

Die Erfindung betrifft ein Verfahren zur kontinuierlichen Herstellung von aromatischen Aminen durch Hydrierung der korrespondierenden Nitroaromaten in Gegenwart von in Reaktionsräumen angeordneten Katalysatoren, bei dem ein adiabat betriebener Reaktionsraum RA einem isotherm betriebenen Reaktionsraum RI nachgeschaltet ist und RA zusätzlich noch über einen separaten Zulauf für den zu hydrierenden Nitroaromaten verfügt, RI von Beginn bis Ende der Hydrierung mit dem zu hydrierenden Nitroaromaten gespeist wird, und das aus RI austretende Produktgemisch von Beginn bis Ende der Hydrierung in RA eingespeist wird, wobei RA zusätzlich über den separaten Zulauf mit dem zu hydrierenden Nitroaromaten gespeist werden kann.

Aromatische Amine sind wichtige Zwischenprodukte, die preiswert und in großen Mengen hergestellt werden müssen. Daher werden Produktionsanlagen für aromatische Amine in der Regel für sehr große Kapazitäten errichtet. Die Hydrierung von Nitroaromaten ist eine stark exotherme Reaktion. Die Abführung und energetische Nutzung der Reaktionswärme ist daher ein wichtiger Punkt bei der Herstellung von Nitroaromaten.

In DE-OS-28 49 002 wird ein Verfahren zur Reduktion von Nitroverbindungen in Gegenwart von ortsfesten, palladiumhaltigen Mehrkomponenten-Trägerkatalysatoren in gekühlten Rohrbündelreaktoren beschrieben. Die Katalysatoren bestehen im Wesentlichen aus 1 g bis 20 g Palladium, 1 g bis 20 g Vanadium und 1 g bis 20 g Blei pro Liter α-Al₂O₃. Nachteilig bei den in der erwähnten Patentliteratur beschriebenen Gasphasenhydrierungen ist die geringe spezifische Belastung der Katalysatoren mit dem zu hydrierenden Nitroaromaten. Die angegebenen Belastungen betragen nur ca. 0.4 kg_{Nitroaromat}/(l_{Katalysator} ·h) bis 0.5 kg_{Nitroaromat}/(l_{Katalysator} h). Die Belastung ist dabei definiert als die Menge an Nitroaromaten in kg, die pro Liter Katalysatorschüttung (Katalysatorvolumina beziehen sich hier und im Folgenden auf das *Schüttvolumen*) innerhalb einer Stunde über den Katalysator geleitet wird. Verbunden mit der geringen Katalysatorbelastung ist eine nicht befriedigende Raum-Zeit-Ausbeute bei großtechnischen Verfahren zur Herstellung von aromatischen Aminen. Ferner sind die Selektivitäten zu Beginn einer Fahrperiode deutlich niedriger als gegen Ende, was zu Ausbeuteverlusten und Problemen bei der Aufarbeitung des Rohprodukts führt.

In der in GB 1 452 466 beschriebenen Verfahrensvariante wird die Hydrierung von Nitroaromaten in thermostatisierten Rohrbündelreaktoren durch einen nachgeschalteten adiabat (d. h. ohne Thermostatisierung) betriebenen Reaktor ergänzt. Als Katalysatoren werden u. a. geträgerte Kupfer- oder Palladium-Katalysatoren eingesetzt. In diesem Verfahren sind der thermostatisierte (d. h. isotherm betriebene) Reaktor und der adiabat betriebene Reaktor, die auch in einem Apparat angeordnet sein können, in Reihe geschaltet, d. h. die aus dem thermostatisierten Reaktor austretende Produktmischung ist die Eduktmischung für den adiabat betriebenen Reaktor. Die Möglichkeit, den adiabat betriebenen Reaktor *zusätzlich* mit Nitrobenzol über eine separate Einspeisung zu beschicken, wird in dieser Schrift nicht offenbart. In bevorzugten Ausführungsformen wird ein unvollständiger Umsatz (z B. nur 70 %) im isotherm betriebenen Teil bewusst in Kauf genommen.

In EP 1 524 259 A1 wird unter anderem ein 2-stufiges Verfahren zur Herstellung von aromatischen Aminen beschrieben, in dem die zweite Verfahrensstufe zur Vervollständigung des Umsatzes dient. Dabei kommt ein adiabat betriebener Reaktor zum Einsatz, der einen katalytisch beschichteten Monolithen als Katalysator enthält. Durch die Verwendung dieses "Nachreaktors" in der zweiten Stufe kann die Standzeit des "Hauptreaktors" (der ersten Stufe) verlängert werden, da mit Hilfe des Nachreaktors noch vollständiger Umsatz erreicht werden kann, wenn im Hauptreaktor bereits Nitroaromat durchgebrochen ist.

Nachteilig an den oben genannten Verfahren ist, dass der Nachreaktor nur dann Nitroaromat zu aromatischem Amin umsetzt, wenn der Umsatz des Hauptreaktors nicht (mehr) vollständig ist. Wird möglichst lange möglichst vollständiger Umsatz im Hauptreaktor angestrebt (EP 1 524 259 A1) bedeutet dies, dass der Nachreaktor während großer Teile der Betriebszeit (nämlich der Vollumsatzphase des Hauptreaktors) ungenutzt bleibt. Dies führt zum Abbau wertvollen Produktes durch Folgereaktionen, welche durch den im Nachreaktor enthaltenen Katalysator katalysiert werden. Wird unvollständiger Umsatz im Hauptreaktor bewusst in Kauf genommen (bestimmte Ausführungsformen von GB 1 452 466), so bedeutet dies, dass der Hauptreaktor von Beginn an überlastet, d. h. mit mehr Nitroaromatem als umgesetzt werden kann beschickt, werden muss. Dies führt zur schnellen Desaktivierung und Schädigung des Katalysators, so dass die Standzeit verringert wird und der Vorteil des Nachreaktors überkompensiert werden kann. Aber auch im ersten Fall, bei dem der Hauptreaktor zunächst mit Vollumsatz betrieben wird, kommt es am Ende der Laufzeit schnell zu sehr hohen Gehalten an nicht umgesetzten Nitroaromaten im Produktstrom des Hauptreaktors und damit zu einer Überlastung des Nachreaktors, dessen Katalysator dann schnell desaktiviert.

Es war daher eine Aufgabe der vorliegenden Erfindung, ein Verfahren zur Herstellung von aromatischen Aminen bereitzustellen, welches die Vorteile einer mindestens zweistufigen Verfahrensführung ausnutzt, ohne die genannten Nachteile des Standes der Technik (vermehrte Nebenproduktbildung, Überlastung und damit Desaktivierung des Katalysators der ersten Stufe) aufzuweisen.

Die Aufgabe wurde gelöst durch ein Verfahren zur kontinuierlichen Herstellung von aromatischen Aminen durch Hydrierung der korrespondierenden Nitroaromaten in Gegenwart von in Reaktionsräumen angeordneten Katalysatoren, bei dem
(i) ein adiabat betriebener Reaktionsraum RA einem isotherm betriebenen Reaktionsraum RI nachgeschaltet ist und RA zusätzlich noch über einen separaten Zulauf für den zu hydrierenden Nitroaromaten verfügt,
(ii) RI von Beginn bis Ende der Hydrierung mit dem zu hydrierenden Nitroaromaten gespeist wird, und das aus RI austretende Produktgemisch von Beginn bis Ende der Hydrierung in RA eingespeist wird,
   wobei
(iii) RA zusätzlich, solange der im aus RI austretenden Produktgemisch gemessene Massenanteil an Nitroaromaten (ω_{NA})^{RI} zwischen 0 ppm und 5000 ppm liegt, über den separaten Zulauf mit dem zu hydrierenden Nitroaromaten gespeist wird.

Unter *Reaktionsraum* wird dabei der Raum verstanden, in welchem die Umsetzung von Nitroaromat (bzw. Zwischenprodukten) mit Wasserstoff zum gewünschten aromatischen Amin stattfindet. Der Reaktionsraum befindet sich in einer technischen Vorrichtung zur Durchführung von chemischen Reaktionen, dem Reaktor. Reaktionsraum und Reaktor können, je nach Konstruktion, auch identisch sein. Der Reaktionsraum kann auch nur einen Teil des Reaktors umfassen. Ebenso ist es möglich, dass, mehrere Reaktionsräume, isotherm und/oder adiabat betrieben, innerhalb eines Reaktors liegen.

*Isotherm* bedeutet im Rahmen der vorliegenden Erfindung, dass mindestens der überwiegende Teil der durch die Reaktion freigesetzten Wärme durch dem Fachmann bekannte technische Vorrichtungen abgeführt wird. Bevorzugt wird die Reaktionswärme vollständig durch technische Vorrichtungen abgeführt.

Dementsprechend bedeutet *adiabat* im Rahmen der vorliegenden Erfindung, dass die Reaktionswärme im adiabat betriebenen Reaktionsraum nicht durch technische Vorrichtungen abgeführt wird. Bei adiabater Betriebsweise wird der Reaktionsraum bevorzugt in besonderer Weise gegen Wärmeverluste isoliert. Wenn Wärmeverluste vernachlässigbar sind, spiegelt sich die Reaktionsenthalpie in der Temperaturdifferenz zwischen Eingangs- und Austrittsstrom quantitativ wider ("adiabater Temperatursprung").

Bevorzugte Reaktoren für den isotherm betriebenen Reaktionsraum RI sind thermostatisierte Rohr- oder Rohrbündelreaktoren. Geeignete Ausführungsformen solcher Reaktoren sind z. B. in DE-AS-2 201 528**,** DE-OS-2 207 166**,** DE-OS-198 06 810**,** EP-B-1 439 901**,** EP-A-1 569 745**,** EP-A-1 590 076**,** EP-A-1 587 612**,** EP-A-1 586 370**,** EP-A-1 627 678 oder DE 202 006 014 116 U1 beschrieben. Bevorzugte Reaktoren für den adiabat betriebenen Reaktionsraum RA sind die in DE 10 2006 035 203, Absätze [0030] bis [0033] beschriebenen, was hiermit als Bestandteil der vorliegenden Offenbarung gilt. Eine weitere mögliche Ausführungsform umfasst ein Verfahren, bei dem der in RA angeordnete Katalysator in einer radial durchströmten Filterkerze vorliegt. Dies kann zum Beispiel erreicht werden, indem der Katalysator in einem Korb gehalten wird, der aus zwei konzentrischen, zylindrischen Siebmänteln mit fluiddurchlässigen Wänden aufgebaut ist. Dabei weist ein Siebmantel einen größeren Radius auf als der andere, der auch als Zentralrohr bezeichnet wird, und der Raum zwischen den Siebmänteln ist der Reaktionsraum. Ein Boden dieses Hohlzylinders ist vorzugsweise komplett dicht verschlossen, während der andere nur bis an das Zentralrohr heran geschlossen ist, das an diesem Ende offen ist. Das Fluid kann nun in radialer Richtung von außen nach innen strömen und dann durch das Zentralrohr abgeführt werden. Alternativ kann das Fluid auch durch das Zentralrohr zugeführt werden und dann in radialer Richtung nach außen strömen, wo es dann abgeführt wird. Befindet sich dieser Reaktionsraum im gleichen Reaktor wie der isotherme Reaktionsraum, so ist er meist auf geeignete Weise mit dem Reaktorauslass verbunden. In einer weiteren Ausführungsform kann sich der Korb aber auch in einer stromabwärts vom isothermen Reaktionsraum gelegenen, gegebenenfalls erweiterten, Rohrleitung oder einem weiteren Reaktor befinden.

Im erfindungsgemäßen Verfahren ist mindestens ein adiabat betriebener Reaktionsraum einem isotherm betriebenen *nachgeschaltet*, d. h. das Verfahren umfasst mindestens zwei in Reihe geschaltete Reaktionsräume. Es ist möglich, jedoch nicht zwingend erforderlich, in die nachgeschalteten Reaktionsräume zusätzlich frischen Wasserstoff oder ein Gemisch aus Wasserstoff und Inertgasen einzuleiten. Der in RA eingespeiste Nitroaromat ist die Summe aus in RI nicht umgesetzten Nitroaromaten und über den separaten Zulauf eingespeisten Nitroaromaten.

Es ist auch vorstellbar, mehr als zwei Reaktionsräume in Reihe zu schalten, also bspw. Kaskaden wie "isotherm - adiabat - adiabat", "isotherm - isotherm - adiabat - adiabat" oder andere Kombinationen. Es werden bevorzugt maximal 10 Reaktionsräume hintereinandergeschaltet, besonders bevorzugt maximal 5, ganz besonders bevorzugt maximal 3, außerordentlich ganz besonders bevorzugt maximal 2. Bevorzugt werden der in Anströmrichtung des Nitroaromaten erste Reaktionsraum einer Kaskade isotherm und der letzte Reaktionsraum dieser Kaskade adiabat betrieben. Die Erfindung wird im Folgenden an der Ausführungsform mit zwei hintereinandergeschalteten Reaktionsräumen näher erläutert. Es ist dem Fachmann ein Leichtes, die Angaben erforderlichenfalls auf Systeme mit mehr als zwei Reaktionsräumen zu übertragen.

Unter *Beginn der Hydrierung* wird der Zeitpunkt verstanden, zu dem zum ersten Mal Nitroaromat und Wasserstoff über mindestens einen der Katalysatoren geleitet werden. Das *Ende der Hydrierung* bezeichnet den Zeitpunkt des kompletten Abschaltens der Zufuhr von Nitroaromaten. Die Hydrierung erfolgt im erfindungsgemäßen Verfahren bevorzugt in der Gasphase. Die Reaktionsführung im isotherm betriebenen Reaktionsraum erfolgt dabei bevorzugt wie in DE 196 51 688 A1, Seite 3, Zeilen 29 bis 56 sowie Seite 4, Zeilen 6 bis 16 beschrieben, was hiermit als Bestandteil der vorliegenden Offenbarung gilt. Es ist jedoch zu beachten, dass der Kreisgasstrom nach Passieren des RI nicht abgekühlt wird, sondern dass diese der Kondensation dienende Abkühlung erst nach Passieren des letzten, adiabaten Reaktionsraums geschieht.

Die Zufuhr des Nitroaromaten in den adiabat betriebenen Reaktionsraum über den separaten Zulauf erfolgt bevorzugt durch Verdüsen des flüssigen Nitroaromaten in einen zusätzlichen Frischwasserstoffstrom oder direkt ins Kreisgas nach RI mittels Zwei- oder Einstoffdüsen. Alternativ kann auch eine Verdampfung in Frischwasserstoff und gegebenenfalls Überhitzung des Nitroaromaten erfolgen, der dann gasförmig zum Kreisgasstrom gegeben wird. Die freiwerdende Reaktionswärme wird in diesem Fall mit dem Kreisgasstrom abtransportiert, so dass dieser einen Temperaturanstieg vollzieht. Wie groß dieser sein darf, ist maßgeblich von der Auslegung der auf RA folgenden Rohrleitungen und Apparate abhängig und begrenzt die zu dosierbare Menge an Nitroaromat zwischen RI und RA. Durch die Nutzung eines gemeinsamen Kreisgassystems erfolgt die Regeneration von RA im gleichen Prozessschritt wie die Regeneration von RI (vgl. DE 196 51 688 A1, Seite 3, Zeilen 40 bis 45), jedoch leicht zeitversetzt, da ein Abbrennen von Ablagerungen in RA erst dann erfolgt, wenn nicht mehr der gesamte Sauerstoff des Regenerier-Gasstroms in RI verbraucht und zu CO₂ abreagiert wird.

Der *Massenanteils an Nitroaromaten* im aus RI austretenden Produktgemisch wird in regelmäßigen Abständen (mindestens alle 48 Stunden) analytisch ermittelt. Hierzu werden an produktführenden Leitungen oder Apparaten aus dem Stand der Technik bekannte Probenahmesysteme installiert. Die analytische Bestimmung des Massenanteils an Nitroaromaten erfolgt mittels Gaschromatographie. Das Produktgemisch umfasst das aromatische Amin, Wasser, Wasserstoff, ggf. Inertgase, ggf. weitere nichtkondensierbare Gase (z. B. Methan aus Verunreinigungen im Wasserstoff) und ggf. nicht umgesetzten Nitroaromaten sowie Nebenprodukte der Reaktion, wie zum Beispiel im Fall der Hydrierung von Nitrobenzol zu Anilin Phenol, Cyclohexylamin, Diphenylamin, Aminophenol, Cyclohexanon, Benzol, etc.

Erfahrungsgemäß wird in der großtechnischen Produktion noch nicht unmittelbar nach Beginn der Hydrierung Vollumsatz an Nitroaromaten erzielt, sondern im Allgemeinen erst nach einer *Anfahrphase* von einigen Stunden. Die genaue Dauer der Anfahrphase ist von den im Einzelfall vorliegenden Bedingungen abhängig. Im Allgemeinen überschreitet die Anfahrphase einen Zeitraum von 12 Stunden nicht. In dieser Anfahrphase liegt der Massenanteil an Nitroaromaten im aus RI austretenden Produktgemisch über 5000 ppm. Durch den in RA angeordneten Katalysator wird dieser Restgehalt an Nitrobenzol in der Anfahrphase reduziert, und zwar auf Werte im aus RA austretenden Produktgemisch von maximal 2000 ppm, bevorzugt maximal 500 ppm, besonders bevorzugt maximal 10 ppm.

Erst wenn der Massenanteil an Nitroaromaten im aus RI austretenden Produktgemisch 5000 ppm erreicht oder unterschreitet (der Reaktor für die Fahrperiode "eingefahren" ist), wird mit der zusätzlichen Einspeisung an Nitroaromaten in RA über den *separaten Zulauf* begonnen. Die Produktionsanlage befindet sich nun in der *Hauptproduktionsphase,* d. i. der Zeitraum zwischen dem Ende der Anfahrphase und dem Beginn der *Endphase* der Hydrierung, d. i. der Zeitraum gegen Ende einer Fahrperiode, in dem - infolge Desaktivierung des Katalysators - die spezifische Belastung schrittweise gesenkt werden muss. Die spezifischen Belastungen an Nitroaromat, mit denen die beiden Reaktionsräume beschickt werden, werden in jeder Produktionsphase so eingestellt, dass der Gesamtumsatz an Nitroaromaten möglichst vollständig ist, d. h. der Massenanteil an Nitroaromat im aus RA austretenden Produktgemisch beträgt maximal 1000 ppm, bevorzugt maximal 500 ppm, besonders bevorzugt maximal 100 ppm, besonders bevorzugt maximal 10 ppm und außerordentlich besonders bevorzugt 0 ppm. Die höheren Werte (> 100 ppm bis 1000 ppm) werden dabei in der Anfahr- und Endphase, die niedrigeren (0 ppm bis ≤ 100 ppm) in der Hauptproduktionsphase erzielt.

Die spezifische Belastung (B_{RI}) des isotherm betriebenen Reaktionsraums mit Nitroaromaten liegt in der Hauptproduktionsphase im Bereich zwischen 0,20 kg_{Nitroaromat}/(l_{Katalysator} · h) und 5,00 kg_{Nitroaromat}/(l_{Katalysator} · h), bevorzugt im Bereich zwischen 0,50 kg_{Nitroaromat}/(l_{Katalysator} · h) und 3,00kg_{Nitroaromat}/(l_{Katalysator} · h), besonders bevorzugt im Bereich zwischen 0,60 kg_{Nitroaromat}/(l_{Katalysator} · h) und 1,50 kg_{Nitroaromat}/(l_{Katalysator} · h). In der Anfahrphase wird B_{RI} schrittweise von Null auf den gewünschten Wert gesteigert, in der Endphase schrittweise vom jeweiligen Wert auf Null gesenkt.

Die spezifische Belastung des adiabat betriebenen Reaktionsraums über den separaten Zulauf (B_{RA})^{sep.} liegt in der Hauptproduktionsphase im Bereich zwischen 0,020 kg_{Nitroaromat}/(l_{Katalysator} · h) und 0,500 kg_{Nitroaromat}/(l_{Katalysator} · h), bevorzugt im Bereich zwischen 0,050 kg_{Nitroaromat}/l_{Katalysator} · h) und 0,300 kg_{Nitroaomat}/(l_{Katalysator} · h), besonders bevorzugt im Bereich zwischen 0,100 kg_{Nitroaromat}/(l_{Katalysator} · h) und 0,200 kg_{N}itroaromat/(lKatalysator . h). In der Anfahrphase beträgt (B_{Ra})^{sep} Null und wird zu Beginn der Hauptproduktionsphase schrittweise auf den gewünschten Wert gesteigert. In der Endphase wird (B_{RA})^{sep.} schrittweise vom jeweiligen Wert auf Null gesenkt.

Der Massenanteil an Nitroaromat, der sich im aus RI bzw. RA austretenden Produktgemisch befindet, kann analytisch mittels Gaschromatographie bestimmt werden. Geeignete Methoden hierzu sind dem Fachmann hinlänglich bekannt. Daneben ist es für den Fachmann ein Leichtes, über die Reaktionsenthalpie der Hydrierung und die Wärmekapazität des Mediums eine Korrelation zwischen dem adiabaten Temperatursprung, der sich in RA (also der Differenz aus der Produktaustrittstemperatur und der Temperatur des Eduktgemisches vor Kontakt mit dem in RA befindlichen Katalysator), und den bekannten Mengen an Nitroaromat, die in RA eingespeist werden (also die Summe aus nicht umgesetzten Nitroaromaten aus RI und über den separaten Zulauf zusätzlich eingespeisten Nitroaromaten) derart herzustellen, dass hieraus der Massenanteil an Nitroaromaten im aus RA austretenden Produktgemisch errechnet werden kann. Umgekehrt kann auch bei analytischer Bestimmung des Massenanteils von Nitroaromat, der aus RA austritt, über den adiabaten Temperatursprung und die zusätzlich eingespeiste Menge an Nitroaromat auf den Anteil an nicht umgesetzten Nitroaromaten aus RI zurückgeschlossen werden. Ein Anstieg der Temperatur des aus RA austretenden Produktgemisches ist häufig ein erstes Anzeichen für einen unzureichenden Umsatz im isotherm betriebenen Reaktionsraum. Eine innerhalb enger Grenzen konstante Temperatur des aus RA austretenden Produktgemisches ist dagegen ein Anzeichen für eine zufriedenstellende Reaktionsführung, die so kontrolliert werden kann, dass der Gesamtumsatz möglichst vollständig (siehe oben) ist.

Sobald der Massenanteil an Nitroaromat im aus RI austretenden Produktgemisch so große Werte annimmt, dass eine Überlastung des Nachreaktors (und damit zu hohe Massenanteile an Nitroaromaten im aus RA austretenden Produktgemisch) zu befürchten ist, wird die spezifische Belastung des adiabat betriebenen Reaktionsraums über den separaten Zulauf (B_{RA})^{sep}. schrittweise auf 0 reduziert und die Reaktion so lange weitergeführt, bis auch mit dem adiabat betriebenen Reaktionsraum kein zufriedenstellender Umsatz mehr erreicht wird. Bevorzugt erfolgt die Reduktion der separaten Einspeisung von Nitroaromaten für den adiabat betriebenen Reaktionsraum in dem Umfang, in dem nicht umgesetzter Nitroaromat aus RI austritt. Als Anhaltspunkt hierfür kann, wie oben erläutert, die Temperatur des aus RA austretenden Produktstroms dienen. Durch den durchbrechenden Nitroaromaten würde sich der adiabate Temperatursprung vergrößern, wenn die zudosierte Menge an Edukten konstant bliebe. Konkret wird dabei bevorzugt so vorgegangen, dass man, sobald (ω_{NA})^{RI} von Werten im Bereich zwischen 0 ppm und 1000 ppm auf Werte über 1000 ppm steigt, die pro Liter Katalysator und Stunde über den separaten Zulauf in RA eingespeiste Menge an Nitroaromaten (= (B_{RA})^{sep.}) schrittweise so reduziert, dass die Temperatur des aus RA austretenden Produktgemisches im Bereich von ± 10 K, bevorzugt im Bereich von ± 5 K, besonders bevorzugt im Bereich von ± 2 K, konstant bleibt. Die Hydrierung wird dann bevorzugt noch so lange weitergeführt, bis auch nach dem adiabat betriebenen Reaktionsraum der Gehalt an Nitroaromat im Produkt ein nicht mehr akzeptables Niveau erreicht. Bevorzugt wird die Hydrierung beendet, sobald der im aus RA austretenden Produktgemisch bestimmte Massenanteil an Nitroaromaten (ω_{NA})^{RA} von Werten im Bereich zwischen 0 ppm und 1000 ppm auf Werte über 1000 ppm steigt. Die Angabe "Liter Katalysator" bezieht sich im Rahmen dieser Erfindung stets auf das Schüttvolumen des Katalysators.

Als Katalysatoren für den isotherm betriebenen Reaktionsraum eignen sich grundsätzlich alle dem Fachmann bekannten Hydrierkatalysatoren. Bevorzugt werden Mehrkomponenten-Trägerkatalysatoren wie in EP-A-0 011 090 Seite 5, Zeile 10 bis Seite 8 Zeile 5 beschrieben, eingesetzt. Dieser Textabschnitt gilt hiermit als Bestandteil der vorliegenden Offenbarung.

Als Katalysatoren für den adiabat betriebenen Reaktionsraum eignen sich grundsätzlich alle dem Fachmann bekannten Hydrierkatalysatoren. Bevorzugt werden Mehrkomponenten-Trägerkatalysatoren in geeigneten Haltevorrichtungen verwendet. Aufgrund des geringen Druckverlusts eignen sich z. B. auf monolithischen Trägern aufgebrachte Katalysatoren wie in EP 1 524 259 A1, Seite 5, Beispiel 1, beschrieben, was hiermit als Bestandteil der vorliegenden Offenbarung gilt. Besonders bevorzugt werden Katalysatoren verwendet, die auf Drahtgestricken (d. h. auf aus Drähten oder Metallfäden gefertigten Maschenwaren) geträgert sind. Die Herstellung der Drahtgestricke erfolgt mit an sich bekannten Drahtverarbeitungsmaschinen, wie sie beispielsweise für Anwendungen in der Automobilindustrie, der Verfahrens- und Umwelttechnik gebräuchlich sind (z. B. Drahtstrickmaschinen). In dieser Ausführungsform betrifft die Erfindung demnach ein Verfahren, bei dem der in RA angeordnete Katalysator katalytisch aktive Komponenten auf einem Drahtgestrick enthält, und bei dem die katalytisch aktiven Komponenten mindestens umfassen:
(a) 1- 00 g/l_{Träger} mindestens eines Metalls der Gruppen 8 bis 12 des Periodensystems der Elemente, und
(b) 0 - 00 g/l_{Träger} mindestens eines Übergangsmetalls der Gruppen 4 bis 6 und 12 des Periodensystems der Elemente, und
(c) 0 - 00 g/l_{Träger} mindestens eines Metalls der Hauptgruppenelemente der Gruppen 14 und 15 des Periodensystems der Elemente.

Durch das erfindungsgemäße Verfahren kann nicht nur die Standzeit des isotherm betriebenen Reaktionsraumes (RI) erhöht werden, sondern auch der adiabat betriebene Reaktionsraum optimal ausgenutzt werden, da er schon vor dem Zeitpunkt, an dem der Umsatz in RI nicht mehr vollständig ist, Nitroaromat unter kontrollierten Bedingungen, d. h. ohne Überlastung, zum aromatischen Amin hydriert. Das Verfahren ist besonders geeignet zur Erweiterung der Produktionskapazität bestehender isotherm arbeitender Anlagen, da die aufwändig konstruierten isotherm betriebenen Reaktoren nicht deinstalliert werden müssen. Insbesondere eignet sich das erfindungsgemäße Verfahren zur Herstellung von Anilin durch Hydrierung von Nitrobenzol.

### Beispiele:

### Beispiel 1

Ein Liter eines α-Al₂O₃-Trägers in Kugelform mit 3 bis 5 mm Durchmesser, einer BET-Oberfläche von 9,8 m²/g, einer Saugfähigkeit von 45,1 ml Wasser pro 100 g Träger und einer Schüttdichte von 812 g/l wurde mit 366 ml einer 10,8 g NaOH enthaltenden wässrigen Lösung imprägniert. Die Lösung wurde innerhalb weniger Minuten völlig vom Träger aufgenommen.

Der feuchte Träger wurde in einem warmen aufsteigenden starken Luftstrom getrocknet. Die Trockenzeit bis zur Gewichtskonstanz betrug ungefähr 15 Minuten. Der Restfeuchtegehalt lag nach dem Abkühlen bei etwa 1 % der Saugfähigkeit des Trägers.

Der so vorbehandelte Träger wurde seiner Saugfähigkeit entsprechend mit 366 ml einer wässrigen Natriumtetrachloropalladat-Lösung, die 9g Palladium enthielt, getränkt und 15 Minuten stehen gelassen. Zur Reduktion der auf dem Träger abgeschiedenen Palladiumverbindung zu metallischem Palladium wurde der Katalysator mit 400 ml einer 10%igen wässrigen Hydrazinhydrat-Lösung überschichtet und 2 Stunden stehen gelassen. Danach wurde der Katalysator mit vollentsalztem Wasser gründlich gespült, bis keine Ionen der bei der Katalysatorherstellung verwendeten Verbindungen im Waschwasser mehr nachweisbar waren, was nach ca. 10 Stunden der Fall war.

Anschließend wurde wieder in einem starken warmen aufsteigenden Luftstrom bis zur Gewichtskonstanz getrocknet. Der Pd-haltige Katalysator wurde anschließend mit 366 ml einer wässrigen, 9 g Vanadium als Vanadyloxalat enthaltenden Lösung getränkt. Die Trocknung des Trägers im Warmluftstrom erfolgte wie oben angegeben. Anschließend wurde der Katalysator in einem Rohrofen bei 300 °C 6 Stunden thermisch behandelt, wobei das Oxalat zersetzt wurde.

Zum Schluss wurde der Katalysator mit 366 ml einer wässrigen, 3 g Blei in Form von Bleiacetat enthaltenden Lösung getränkt und wiederum im Aufsteigenden Luftstrom getrocknet.

Der fertige Katalysator enthielt 9 g Palladium, 9 g Vanadium und 3 g Blei und entsprach dem Katalysator aus DE 2849002.

### Beispiel 2 (Vergleichsbeispiel)

In ein mit Öl thermostatisiertes Reaktorrohr (isotherm betriebener Reaktionsraum) mit einem Innendurchmesser von ca. 26 mm wurde eine 285 cm hohe Schüttung eines Katalysators, hergestellt gemäß Beispiel 1, gefüllt. Der Katalysator wurde zuerst mit Stickstoff, dann mit Wasserstoff gespült und anschließend in einem Wasserstoffstrom von ca. 15701/h in 5 Stunden auf 240 °C aufgeheizt. Dann wurde begonnen, Nitrobenzol im Wasserstoffstrom zu verdampfen. Die spezifische Belastung wurde schrittweise auf 1,02 kg_{Nitrobenzol}/(l_{Katalysator} · h) erhöht, wobei darauf geachtet wurde, dass die Temperatur im Reaktionsrohr an keiner Stelle über 450 °C anstieg. Im zeitlichen Mittel betrug die spezifische Belastung damit 1,00 kg_{Nitrobenzol}/(l_{Katalysator} · h). Die Temperatur in der Katalysatorschüttung (und damit die Lage der Reaktionsfront) wurde verfolgt und gegen Ende der Reaktion, ab ca. 700 Stunden, wurde die Öltemperatur schrittweise von 240 °C auf 300 °C erhöht, so dass möglichst lange Vollumsatz erreicht wurde. Nach 1020 h konnten im Reaktionsprodukt mehr als 1000 Massen-ppm Nitrobenzol detektiert werden und die Reaktion wurde beendet. Es wurden ca. 1500 kg Nitrobenzol umgesetzt. Die durchschnittliche Selektivität betrug 99,0 %.

### Beispiel 3 (Erfindungsgemäßes Beispiel)

Zusätzlich zum Versuchsaufbau in Beispiel 2 wurde dem thermostatisierten Rohrreaktor ein adiabat betriebener Reaktor (adiabat betriebener Reaktionsraum, "Nachreaktor") nachgeschaltet. Dieser hatte ein Volumen von 250 ml und wurde mit einem auf Drahtgestrick geträgerten Katalysator beschickt. Die katalytisch aktive Beschichtung des Gestricks bestand aus ca. 75 g/l Aluminiumoxid und je 4 g/l Palladium und Vanadium, die nacheinander aufgebracht wurden. Zwischen den beiden Reaktoren wurde eine separate Einspeisung von Nitrobenzol und Wasserstoff in den Produktstrom vorgesehen.

Die Reaktoren wurden zunächst mit Stickstoff, dann mit Wasserstoff gespült und anschließend in einem Wasserstoffstrom von ca. 1570 Nl/h in 5 Stunden auf 240 °C aufgeheizt. Dann wurde begonnen, Nitrobenzol im Wasserstoffstrom zu verdampfen. Die spezifische Belastung wurde schrittweise auf 1,02 kg_{Nitrobenzol}/(l_{Katalysator} · h) erhöht, wobei darauf geachtet wurde, dass die Temperatur im Reaktionsrohr an keiner Stelle über 450 °C anstieg.

Gleichzeitig wurden über den separaten Zulauf 90 g/h Nitrobenzol und 50 l/h Wasserstoff zwischen dem Haupt- und dem Nachreaktor zudosiert.

Die Temperatur in der Katalysatorschüttung des isotherm betriebenen Reaktionsraumes ("Hauptreaktor") - und damit die Lage der Reaktionsfront - wurde verfolgt und gegen Ende der Reaktion, ab ca. 700 Stunden, wurde die Öltemperatur schrittweise von 240 °C auf 300 °C erhöht, so dass möglichst lange Vollumsatz im Hauptreaktor erreicht wurde. Nach etwa 1000 h stieg der Nitrobenzolgehalt im Produktaustritt des isotherm betriebenen Reaktionsraumes auf Werte > 1000 Massen-ppm. Ab diesem Zeitpunkt wurde die Belastung des Nachreaktors langsam bis auf 0 kg_{Nitrobenzol}/(l_{Katalysator} · h) abgesenkt, so dass die Produktaustrittstemperatur innerhalb eines Bereiches von ± 2 K konstant gehalten wurde. Nach 1080 h stieg auch der Nitrobenzolgehalt im Produktaustritt des adiabat betriebenen Reaktionsraumes auf Werte > 1000 Massen-ppm, und die Reaktion wurde beendet. Insgesamt wurden so in diesem Zyklus ca. 1600 kg Nitrobenzol im Hauptreaktor und ca. 95 kg Nitrobenzol im Nachreaktor, also insgesamt ca. 1695 kg Nitrobenzol umgesetzt. Es konnte also sowohl die Ausnutzung des Hauptreaktors verbessert werden als auch während der Vollumsatz-Phase eine erhöhte Menge Nitrobenzol in dieser Versuchsanordnung umgesetzt werden.

## Patentansprüche

1. Verfahren zur kontinuierlichen Herstellung von aromatischen Aminen durch Hydrierung der korrespondierenden Nitroaromaten in Gegenwart von in Reaktionsräumen angeordneten Katalysatoren, bei dem
(i) ein adiabat betriebener Reaktionsraum RA einem isotherm betriebenen Reaktionsraum RI nachgeschaltet ist und RA zusätzlich noch über einen separaten Zulauf für den zu hydrierenden Nitroaromaten verfügt,
(ii) RI von Beginn bis Ende der Hydrierung mit dem zu hydrierenden Nitroaromaten gespeist wird, und das aus RI austretende Produktgemisch von Beginn bis Ende der Hydrierung in RA eingespeist wird,
wobei
(iii) RA zusätzlich, solange der im aus RI austretenden Produktgemisch gemessene Massenanteil an Nitroaromaten (ω_{NA})^{RI} zwischen 0 ppm und 5000 ppm liegt, über den separaten Zulauf mit dem zu hydrierenden Nitroaromaten gespeist wird.

2. Verfahren nach Anspruch 1, bei dem, sobald (ω_{NA})^{RI} von Werten im Bereich zwischen 0 ppm und 1000 ppm auf Werte über 1000 ppm steigt, die pro Liter Katalysator und Stunde über den separaten Zulauf in RA eingespeiste Menge an Nitroaromaten schrittweise so reduziert wird, dass die Temperatur des aus RA austretenden Produktgemisches im Bereich von ± 10 K konstant bleibt.

3. Verfahren nach Anspruch 1, bei dem die Hydrierung beendet wird, sobald der im aus RA austretenden Produktgemisch bestimmte Massenanteil an Nitroaromaten (ω_{NA})^{RA} von Werten im Bereich zwischen 0 ppm und 1000 ppm auf Werte über 1000 ppm steigt.

4. Verfahren nach Anspruch 1, bei dem der in RA angeordnete Katalysator katalytisch aktive Komponenten auf einem Drahtgestrick enthält, und bei dem die katalytisch aktiven Komponenten mindestens umfassen:
(a) 1-100 g/l_{Träger} mindestens eines Metalls der Gruppen 8 bis 12 des Periodensystems der Elemente, und
(b) 0-100 g/l_{Träger} mindestens eines Übergangsmetalls der Gruppen 4 bis 6 und 12 des Periodensystems der Elemente, und
(c) 0 - 00 g/l_{Träger} mindestens eines Metalls der Hauptgruppenelemente der Gruppen 14 und 15 des Periodensystems der Elemente.

5. Verfahren nach Anspruch 1, bei dem der in RA angeordnete Katalysator in einer radial durchströmten Filterkerze vorliegt.

6. Verfahren nach Anspruch 1, bei dem als Nitroaromat Nitrobenzol eingesetzt wird.

## Claims

1. Process for the continuous preparation of aromatic amines by hydrogenation of the corresponding nitroaromatics in the presence of catalysts arranged in reaction spaces, in which
(i) an adiabatically operated reaction space RA is connected downstream of an isothermally operated reaction space RI and RA additionally also has a separate feed for the nitroaromatic to be hydrogenated,
(ii) RI is fed with the nitroaromatic to be hydrogenated from the start to the end of the hydrogenation, and the product mixture emerging from RI is fed into RA from the start to the end of the hydrogenation.
wherein
(iii)RA additionally, as long as the weight content of nitroaromatic measured in the product mixture emerging from RI (ω_{NA})^{RI} is between 0 ppm and 5,000 ppm, is fed via the separate feed with the nitroaromatic to be hydrogenated.

2. Process according to claim 1, in which as soon as (ω_{NA})^{RI} increases from values in the range between 0 ppm and 1,000 ppm to values above 1,000 ppm, the amount of nitroaromatic fed into RA via the separate feed per litre of catalyst and hour is reduced stepwise such that the temperature of the product mixture emerging from RA remains constant in the range of ± 10 K.

3. Process according to claim 1, in which the hydrogenation is ended as soon as the weight content of nitroaromatic determined in the product mixture emerging from RA (ω_{NA})^{RA} increases from values in the range between 0 ppm and 1,000 ppm to values above 1,000 ppm.

4. Process according to claim 1, in which the catalyst arranged in RA contains catalytically active components on a knitted wire fabric, and in which the catalytically active components comprise at least:
(a) 1 - 100 g/lₛᵤₚₚₒᵣₜ of at least one metal of groups 8 to 12 of the periodic table of the elements, and
(b) 0 - 100 g/lₛᵤₚₚₒᵣₜ of at least one transition metal of groups 4 to 6 and 12 of the periodic table of the elements, and
(c) 0 - 100 g/lₛᵤₚₚₒᵣₜ of at least one metal of the main group elements of groups 14 and 15 of the periodic table of the elements.

5. Process according to claim 1, in which the catalyst arranged in RA is present in a filter candle flowed through radially.

6. Process according to claim 1, in which nitrobenzene is employed as the nitroaromatic.

## Revendications

1. Procédé de fabrication continue d'amines aromatiques par hydrogénation des composés nitro-aromatiques correspondants en présence de catalyseurs agencés dans des chambres de réaction, dans lequel
(i) une chambre de réaction RA exploitée de manière adiabatique est connectée en aval d'une chambre de réaction RI exploitée de manière isotherme, et RA dispose en outre d'une alimentation séparée pour les composés nitro-aromatiques à hydrogéner,
(ii)RI est alimentée avec les composés nitro-aromatiques à hydrogéner du début à la fin de l'hydrogénation, et le mélange de produits sortant de RI est introduit dans RA du début à la fin de l'hydrogénation,
(iii) RA étant également alimentée avec les composés nitro-aromatiques à hydrogéner par l'alimentation séparée tant que la proportion en masse de composés nitro-aromatiques (ω_{NA})^{RI} mesurée dans le mélange de produits sortant de RI est comprise entre 0 ppm et 5 000 ppm.

2. Procédé selon la revendication 1, dans lequel, dès que (ω_{NA})^{RI} augmente à partir de valeurs dans la plage comprise entre 0 ppm et 1 000 ppm à des valeurs supérieures à 1 000 ppm, la quantité de composés nitro-aromatiques introduite dans RA par l'alimentation séparée par litre de catalyseur et par heure est réduite graduellement de sorte que la température du mélange de produits sortant de RA reste constante dans la plage de ± 10 K.

3. Procédé selon la revendication 1, dans lequel l'hydrogénation est terminée dès la proportion en masse de composés nitro-aromatiques (ω_{NA})^{RA} déterminée dans le mélange de produits sortant de RA augmente à partir de valeurs dans la plage comprise entre 0 ppm et 1 000 ppm à des valeurs supérieures à 1 000 ppm.

4. Procédé selon la revendication 1, dans lequel le catalyseur agencé dans RA contient des composants catalytiquement actifs sur un tricot métallique et dans lequel les composants catalytiquement actifs comprennent au moins :
(a) 1 à 100 g/lₛᵤₚₚₒᵣₜ d'au moins un métal des groupes 8 à 12 du tableau périodique des éléments et
(b) 0 à 100 g/lₛᵤₚₚₒᵣₜ d'au moins un métal de transition des groupes 4 à 6 et 12 du tableau périodique des éléments et
(c) 0 à 100 g/lₛᵤₚₚₒᵣₜ d'au moins un métal des éléments des groupes principaux des groupes 14 et 15 du tableau périodique des éléments.

5. Procédé selon la revendication 1, dans lequel le catalyseur agencé dans RA se trouve dans une cartouche de filtration traversée radialement.

6. Procédé selon la revendication 1, dans lequel le nitrobenzène est utilisé en tant que composé nitro-aromatique.
